Europäisches Patentamt

(19) European Patent Office

Office européen des brevets

(11) Publication number: **0 009 808**

**A1**

(12) # EUROPEAN PATENT APPLICATION

(21) Application number: **79103766.6**

(22) Date of filing: **03.10.79**

(51) Int. Cl.³: **A 61 K 31/645**
//(A61K31/645, 31/60)

(30) Priority: **04.10.78 US 948445**

(43) Date of publication of application:
**16.04.80 Bulletin 80/8**

(84) Designated Contracting States:
**AT BE CH DE FR GB IT LU NL SE**

(71) Applicant: **Merck & Co., Inc.**
**Patent Department 126 East Lincoln Avenue**
**Rahway, N.J. 07065(US)**

(72) Inventor: **Bayne, Gilbert M.**
**208 Woods Road**
**Glenside, Pennsylvania 19038(US)**

(74) Representative: **Abitz, Dr.-Ing. Walter et al,**
**Abitz, Morf, Gritschneder P.O. Box 86 01 09**
**8000 Munich 86(DE)**

(54) **Composition having skeletal muscle relaxant activity.**

(57) A combination of cyclobenzaprine and diflunisal is useful for inducing skeletal muscle relaxation.

BAD ORIGINAL

EP 0 009 808 A1

Croydon Printing Company Ltd.

- 1 -                              16201

COMPOSITION HAVING SKELETAL MUSCLE RELAXANT ACTIVITY

This invention relates to compositions for treating muscle spasm of local origin. More particularly, the invention is concerned with a beneficial integration of cyclobenzaprine and diflunisal, 5-(2,4-difluorophenyl)salicylic acid, in the management of skeletal muscle spasm.

Muscle spasm and related disorders affecting the skeletal muscles affect a large segment of the population and are associated with such conditions as herniated intervertebral discs, muscular and ligamentous sprains and strains, whiplash injury, fractures, dislocations, arthritis, cramps, sciatica, low-back disorders, and the like. At present a variety of medicinals are used in an attempt to alleviate the muscle spasm and the pain associated therewith but use of these various materials unfortunately is limited by concomitant side effects and toxicity which restricts the practical dosage of the materials. Accordingly, there is a continuing search for a medication which will have sufficient potency in tolerated doses to interrupt this pain-spasm cycle in a majority of cases when administered either by the oral or parenteral route.

Further, since the two compounds selected both have a long duration of action, a bedtime dose will maintain this combined effect on pain and spasm throughout a night's sleep of normal duration.

Cyclobenzaprine, or a pharmaceutically acceptable salt thereof, has been found to be useful as a skeletal muscle relaxant and is the subject of U.S. Patent 3,882,246, directed to this discovery. Cyclobenzaprine is highly effective in the treatment of muscular spasm and other similar muscular disorders associated with or caused by injury or arising spontaneously with no known cause.

Diflunisal is a powerful non-addicting analgesic agent with antiinflammatory properties.

This invention relates to a beneficial integration of cyclobenzaprine and diflunisal in the management of acute and chronic musculo-skeletal disorders in which inflammation, pain, spasm and spasticity are components of the symptom complex. These syndromes include bursitis, synovitis, tenosynovitis, fibrositis (myositis, fibro-myositis, lumbago), strains and sprains, muscle trauma, torticollis, whiplash injury and exacerbations of rheumatoid arthritis.

The components of the composition are mutually complementary in their relationship and pharmacologic action, and each has a long duration of action. Of particular importance is that, in accordance with the invention, clinical dosages of both components may be employed in a twice a day regimen rather than three to four times a day in the management of skeletal muscle hyperactivity manifest by pain, spasm, spasticity and inflammation.

In a symptom complex involving pain and spasm either component of the complex can trigger the other thus: pain ⟷ spasm. Elimination of one component is beneficial, but elimination of both components is optimal. Even with a highly effective drug like cyclobenzaprine the practical dosage of the drug is limited by side effects such as dryness of the mouth and drowsiness, so that with patients with moderate to severe symptoms of disease the relief of symptoms is incomplete in most patients. Similarly, with diflunisal the benefit obtained with tolerable doses is incomplete. Through the use of both drugs there will be sufficient interaction to have an effect on duration of symptoms not seen with either alone resulting from an interruption of the self-perpetrating cycle of pain and spasm. Accordingly, this invention has the following attribute: employment of complementary drugs to break the pain ⟷ spasm cycle concomitantly, and, thereby to shorten the period of treatment required.

For the purpose of inducing skeletal muscle relaxation and pain relief the compounds of the invention may be administered orally, parenterally, or rectally in formulations containing conventional non-toxic pharmaceutically acceptable carriers, adjuvants and vehicles. The term parenteral as used herein includes intravenous or intramuscular techniques. In addition to the treatment of warm- blooded animals such as mice, rats, horses, dogs, cats, etc., the combination of the invention is effective in the treatment of humans. It should be noted that the cyclobenzaprine and diflunisal may be administered in combination, i.e., in physical combination in a single unitary dosage form; simultaneously, each in a separate dosage form; successively, i.e., a dosage of cyclobenzaprine followed by a dosage of diflunisal by the same or different route of administration.

As indicated previously it is now possible in accordance with this invention to reduce the usual dose of cyclobenzaprine and yet retain the full therapeutic benefits. Cyclobenzaprine by itself would conventionally be employed as a muscle relaxant at oral dose levels of 0.1 - 1.0 mg./kg. of body weight per day. The optimum dose for an adult human would vary from about 20 mg. to 60 mg. per day. Diflunisal is administered at about 500 mg. to 2000 mg. per day. The enhanced pharmacological activity of the combination is evident over a fairly wide range of proportions of the two principal ingredients. In general, the range of ratios of parts by weight may vary from 10 to 200 parts of diflunisal to one part by weight of cyclobenzaprine; preferably from 35 to 100 parts by weight of the former per part by weight of the latter. Comparable dosages are used in parenteral or rectal administration.

The amount of active ingredient that may be combined with the carrier materials to produce a single dosage form will vary depending upon the host treated and the particular mode of administration. For example, a formulation intended for the oral administration of humans may contain from 5 mg. to 20 mg. of cyclobenzaprine combined with 250 mg. to 1000 mg. of diflunisal compounded with an appropriate and convenient amount of carrier material which may vary from about 5 to about 80 percent of the total composition. Examples of formulations that may be administered on a b.i.d., t.i.d. or q.i.d. basis include cyclobenzaprine 5 mg.; diflunisal 250 mg. and cyclobenzaprine 10 mg.; diflunisal 500 mg.

The pharmaceutical compositions containing the active ingredient may be in a form suitable for oral use, for example, as tablets, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide a pharmaceutically elegant and palatable preparation. Tablets contain the active ingredient in admixture with non-toxic pharmaceutically acceptable excipients which are suitable for the manufacture of tablets. These excipients may be, for example, inert diluents, such as pregelatinized starch, calcium sulfate, microcrystalline cellulose, lactose, fumed silica calcium phosphate or granulating or disintegrating agents, for example, hydroxypropyl cellulose, methyl cellulose, cellulose acetate phthalate, guar gum, ethyl cellulose, maize starch, or alginic acid; binding agents, for example starch, gelatine or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate alone or with a wax may be employed.

Formulations for oral use may also be presented as hard gelatine capsules wherein the active ingredient is mixed with an inert solid diluent, for example, lactose, pregelatinized starch, calcium carbonate, calcium phosphate starch, or kaolin, or as soft gelatine capsules wherein the active ingredient is mixed with water or an oil medium, for example arachis oil, peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropyl cellulose, hydroxypropylmethylcellulose, sodium alginate, polyvinylpyrrolidone, xanthan gum,

- 9 -                          16201

agenous suspenion. This suspension may be formulated
according to the known art using those suitable
dispersing or wetting agents and suspending agents
which have been mentioned above. The sterile
injectable preparation may also be a sterile
injectable solution or suspension in a non-toxic
parenterally-acceptable diluent or solvent, for
example as a solution in water, ethanol or polyethylene glycol. Among the acceptable vehicles and
solvents that may be employed are water, high molecular weight sugar solutions and isotonic sodium
chloride solution. In addition, sterile, fixed oils
are conventionally employed as a solvent or suspending
medium. For this purpose any bland fixed oil may
be employed including synthetic mono- or diglycerides.
In addition, fatty acids such as oleic acid
find use in the preparation of injectibles.

For parenteral administration aqueous
fluid unit dosage forms can be prepared in a variety
of forms such as: a combination of an aqueous
solution of cyclobenzaprine and an aqueous suspension
of diflunisal; a combination of any aqueous solution
of cyclobenzaprine and aqueous solution of a
diflunisal salt such as the choline salt; an oil
suspension of cyclobenzaprine and an oil suspension
of diflunisal; a freeze dried mixture of cyclobenzaprine and diflunisal choline salt; and an
aqueous suspension of diflunisal and a separate
aqueous solution of cyclobenzaprine to be combined
at time of use.

For veterinary oral use the active
ingredient is conveniently prepared in the
form of a food premix. The food premix can comprise or sorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soya bean lecithin, and esters or partial esters derived from fatty acids and hexitol anhydrides, for example sorbitan mono-oleate, and condensation products of the partial esters with ethylene oxide, for example polyoxyethylene sorbitan mono-oleate. The emulsions may also contain sweetening and flavoring agents.

Syrups and elixirs may be formulated with sweetening agents, for example glycerol, sorbitol or sucrose. Such formulations may also contain a demulcent, a preservative and flavoring, solubilizing and coloring agents.

The pharmaceutical compositions may be in the form of a sterile injectable preparation, for example as a sterile injectable aqueous or ole- therapeutic use in humans and animals, as disclosed in detail in this specification, these being features of the present invention. Examples of suitable unit dosage forms in accord with this invention are tablets, capsules, pills, powder packets, granules, segregated multiples of any of the foregoing, and other forms as herein described.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy. In general, the dosage regimen in carrying out the methods of this invention is that which insures maximum therapeutic response until improvement is obtained and thereafter is the minimum effective level which continues to provide relief.

This invention is illustrated by the following examples:

### EXAMPLE 1

|  | Per tablet, mg. |
|---|---|
| Cyclobenzaprine | 10.0 |
| Diflunisal | 500.0 |
| Lactose | 79.0 |
| Starch, corn | 65.0 |
| Hydroxypropyl cellulose (as 2% in ethanol) | 8.0 |

                                          Per tablet, mg.

Add:

    Starch, corn                    55.0

    Guar gum                        55.0

    Magnesium stearate               4.0


The cyclobenzaprine, diflunisal, lactose and corn starch are reduced to a fine powder by milling and remixing. The mixture is granulated with the hydroxypropyl cellulose solution. The wetted mass is passed through a No. 10 stainless steel screen and dried in the dark at 110°F. The dried granules are passed through a No. 20 stainless steel screen, and the additional quantity of corn starch, guar gum and magnesium stearate added. The mixture is compressed using a 1/2" standard curvature punch into tablets and the tablet may be coated with a conventional protective film containing various types of cellulose polymers, dyes and opacifying agents.


### EXAMPLE 2

                                          Per tablet, mg.

Cyclobenzaprine                              8.0

Diflunisal                                 400.0

Calcium phosphate, dibasic                 100.0

Lactose                                     90.0

Ethyl cellulose (as 5% in
  ethanol)                                  4.0


Add:

    Starch, corn                    25.0

    Magnesium stearate               4.0

The cyclobenzaprine, diflunisal, calcium phosphate and lactose are mixed to a fine powder, i.e., through 60 mesh. The powder is granulated with the ethyl cellulose solution and the moist mass passed through a No. 10 screen, dried at 110°F. and rescreened through a No. 20 stainless steel screen. The corn starch and magnesium stearate are added and the mixture compressed to tablets with an 1/2" punch. These tablets may be coated with a protective film.

### EXAMPLE 3

|  | Per tablet, mg. |
|---|---|
| Cyclobenzaprine | 10.0 |
| Diflunisal | 600.0 |
| Lactose | 187.0 |
| Starch, corn | 55.00 |
| Hydroxypropyl cellulose (2% in ethanol) | 4.00 |

Add:

| Starch, corn | 55.00 |
|---|---|
| Guar gum | 55.00 |
| Magnesium stearate | 4.00 |

The tablets are prepared as described in Example 1.

### EXAMPLE 4

|  | Per tablet, mg. |
|---|---|
| Cyclobenzaprine | 8.0 |
| Diflunisal | 500.0 |
| Pregelatinized starch | 210.0 |
| Hydroxypropyl cellulose | 17.0 |

Per tablet, mg.

Add:

  Microcrystalline

  cellulose      88.0

  Magnesium stearate   8.0

  The tablets are prepared as described in Example 1.


## EXAMPLE 5

|  | Per tablet, mg. |
|---|---|
| Cyclobenzaprine | 5.0 |
| Diflunisal | 600.0 |
| Calcium phosphate, dibasic | 180.0 |
| Magnesium stearate | 4.0 |

Add:

| | |
|---|---|
| Corn starch | 55.0 |
| Magnesium stearate | 4.0 |

  The tablets are prepared as in Example 1.

  Examples of capsules containing cyclobenzaprine and diflunisal follow:


## EXAMPLE 6

|  | Per capsule, mg. |
|---|---|
| Cyclobenzaprine | 5.0 |
| Diflunisal | 250.00 |
| Lactose | 415.25 |
| Magnesium stearate | 2.00 |

  The mixed powder is encapsulated.

## EXAMPLE 7

|                      | Per capsule, mg. |
| -------------------- | ---------------- |
| Cyclobenzaprine      | 10.0             |
| Diflunisal           | 300.0            |
| Lactose              | 250.00           |
| Magnesium stearate   | 1.00             |

The powdered components are mixed and put into an opaque gelatin capsule.

## EXAMPLE 8

The following represents an example of sterile aqueous suspensions of the therapeutic mixtures described above:

|                                  | Per ml., mg. |
| -------------------------------- | ------------ |
| Cyclobenzaprine                  | 5.00         |
| Diflunisal                       | 125.00       |
| Polysorbate 80                   | 0.05         |
| Sodium CMC                       | 15.00        |
| Sodium chloride to render isoosmotic |          |
| Benzyl alcohol                   | 9.0          |
| Water for injection to make      | 1.00 ml.     |

The Polysorbate 80 is dispersed at 65°C. in water containing the sodium chloride and benzyl alcohol. The resulting mixture is sterilized by autoclaving. After the vehicle has cooled to room temperature sterilized cyclobenzaprine and diflunisal are dispersed therein. The resulting suspension is subdivided into amber vials with protection from light during the subdivision.

WHAT IS CLAIMED IS:

1. A composition having skeletal muscle relaxant activity comprising a therapeutically effective amount of cyclobenzaprine or a pharmaceutically acceptable non-toxic acid addition salt thereof and diflunisal.

2. The composition of Claim 1 wherein there is present for each part by weight of cyclobenzaprine, from about 10 to 200 parts by weight of diflunisal.

3. The composition of Claim 2 wherein the ratio of cyclobenzaprine to diflunisal is 35-100 parts of the latter per part by weight of the former.

## Claims for Austria

1. Process for preparing a composition having skeletal muscle relaxant activity, characterized by mixing a therapeutically effective amount of cyclobenzaprine or a pharmaceutically acceptable non-toxic acid addition salt thereof and diflunisal.

2. Process according to claim 1 wherein there is used for each part by weight of cyclobenzaprine, from about 10 to 200 parts by weight of diflunisal.

3. Process according to claim 2 wherein there is used a ratio of cyclobenzaprine to diflunisal of 35-100 parts of the latter per part by weight of the former.

European Patent Office

**EUROPEAN SEARCH REPORT**

0009808

Application number

EP 79 10 3766

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| | NL - A - 72 01 002 (MERCK & CO. INC.)<br>  * Claims 1,4,5; page 13 *<br>& GB - A - 1 334 326 (MERCK & CO. INC.)<br><br>----- | 1 | A 61 K 31/645//<br>(A 61 K 31/645<br>A 61 K 31/60) |

TECHNICAL FIELDS SEARCHED (Int.Cl. ³)

A 61 K 31/645

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 07-01-1980 | BRINKMANN |

EPO Form 1503.1  08.78